# EUROPEAN PATENT SPECIFICATION

(11) **EP 1 767 626 B1**
(45) Date of publication and mention of the grant of the patent: **25.03.2009**
(21) Application number: 05748996.5
(22) Date of filing: 07.06.2005
(51) Int. Cl.: C12N 15/09, A01H 5/00

(54) **PERORAL VACCINE CARRIER SYSTEM**
PERORALES IMPFSTOFFTRÄGERSYSTEM
SYSTEME VECTEUR DE VACCIN PERORAL

(30) Priority: 07.06.2004 JP 2004169259
(43) Date of publication of application: 28.03.2007
(73) Proprietor: National Institute of Advanced Industrial Science and Technology, Tokyo 100-8921 (JP); Frontier Science Co. Ltd., Hokkaido 0613241 (JP); THE KITASATO INSTITUTE, Tokyo 108-8642 (JP); THE HOKUREN FEDERATION OF AGRICULTURAL COOPERATIVES, Sapporo-shi Hokkaido, 0608651 (JP)
(72) Inventor: MATSUMURA, T., c/o Hokkaido Center, National Inst., Sapporo-shi, Hokkaido 0628517 (JP); ITO, Akira, The Kitasato Institute, Kitamoto-shi, Saitama 3640026 (JP); KOBAYASHI, Shigeki, The Kitasato Institute, Kitamoto-shi, Saitama 3640026 (JP); KUME, Katsumi, The Kitasato Institute, Kitamoto-shi, Saitama 3640026 (JP); ITO, Keizo, Frontier Science Co., Ltd., Ishikari-shi, Hokkaido 0613241 (JP); ITCHODA, Noriko The Hokuren Federation of, jyo Cyou-ku Sapporo-shi,Hokkaido 0608651 (JP); SUGIMOTO, Chihiro, Obihiro University, Inada-cho, Obihiro-shi, Hokkaido 0808555 (JP)
(74) Representative: Ebner von Eschenbach, Jennifer
(86) International application number: PCT/JP2005/010387
(87) International publication number: WO 2006/008881

(56) References cited:
- WO-A1-00/25574
- WO-A1-00/25574
- JP-A- 07 284 392
- JP-A- 09 508 786
- TACKET C O ET AL: "Human Immune Responses to a Novel Norwalk Virus Vaccine Delivered in Transgenic Potatoes" JOURNAL OF INFECTIOUS DISEASES, CHICAGO, IL, US, vol. 182, 6 July 2000 (2000-07-06), pages 302-305, XP003000315 ISSN: 0022-1899
- MURAMATSU K.: 'Shokubutsu no Idenshi Kumikae Gijutsu o Riyo shita Niwatori Genchubyo Keiko Vaccine Sozai no Kaihatsu' BRAIN TECHNO NEWS vol. 107, 2005, pages 16 - 20, XP002997701
- HUANG Z. ET AL: 'Virus-like particle expression and assembly in plants: hepatitis B and Norwalk viruses' VACCINE vol. 23, no. 15, March 2005, pages 1851 - 1858, XP004768068
- MASON H.S. ETG AL: 'Expression of Norwalk virus capsid protein in transgenic tobacco and potato and its oral immunogenicity in mice' PROC.NATL.ACAD.SCI. USA vol. 93, no. 11, 1996, pages 5335 - 5340, XP002025358
- RICHTER L.J. ET AL: 'Production of hepatitis B surface antigen in transgenic plants for oral immunization' BAT. BIOTECHNOL. vol. 18, no. 11, 2000, pages 1167 - 1171, XP002997702
- SOMEYA Y. ET AL: 'Complete nucleotide sequence of the chiba virus genome and functional expression of the 3C-like protease in Escherichia coli' VIROLOGY vol. 278, no. 2, 2000, pages 490 - 500, XP002236228
- MASON H.S. ET AL: 'Subunit vaccines produced and delivered in transgenic plants as "edible vaccines"' RES.IMMUNOL. vol. 149, no. 1, 1998, pages 71 - 74, XP001084561

## Description

### TECHNICAL FIELD

The present invention relates to a carrier system in which a recombinant plant expressing a target peptide is orally administered to thereby transport the peptide to a target site in the body and cause its function to be exerted, and to the recombinant plant, and relates more particularly to a carrier system in which a plant expressing a fused gene consisting of a gene coding for a target protein and a gene for the Norwalk virus coat protein is orally administered to thereby cause the function of the target protein to be exerted inside the body, and to a recombinant plant.

The present invention relates to a new technique for creating a recombinant plant expressing a target peptide and causing its function to be exerted inside the body by means of oral ingestion, and creates a new technique for producing antibodies by ingestion of a plant in the technical field of human and animal disease prevention by vaccine for example, which is an important basic technology for future progress and development of new techniques in the medical, pharmaceutical and other fields.

The present invention allows the construction of a novel peroral vaccine carrier system capable for example of efficiently preventing leucocytozoon disease of chickens, a hemorrhagic and anemic disease of chickens which was first reported in 1909 to be caused by infection with the hemosporidium (protozoan) Leucocytozoon caulleryi, and which is one of the serious problems in the poultry industry because it leads to lower egg-laying rates, lower rates of gain in weight and higher death rates.

In this system, infection can be effectively prevented when a genetically recombinant plant expressing an antigen is administered orally together with feed, thereby inducing antibodies to the pathogen. The present invention is useful because it can be applied to humans or animals, allowing efficient administration without the stress caused by intramuscular injection, and because it can provide an oral vaccine at low cost since a plant carrying the antibody can be mass produced.

### BACKGROUND ART

The peptide of the present invention includes a wide range of immunogenic peptides, functional peptides and the like, but one that is known is an immunogenic peptide from a Leucocytozoon protozoan that infects chickens. Chicken Leucocytozoon disease is a disease caused by infection with haemosporidium Leucocytozoon caulleryi. This protozoan is disseminated biologically by means of the midge Culicoides arakawae belonging to Ceratopogonidae of Diptera, a species of blood-sucking insect. The life cycle of Leucocytozoon caulleryi is fairly well known, involving three developmental stages: schizogony and gametogony in the body of the chicken host and sporogony in the body of the carrier Culicoides arakawae (hereunder called as Culicoides).

When a Culicoides infected with the protozoan sucks blood, sporozoites infiltrate the chicken's body together with the Culicoides saliva, parasitizing cells of the vascular endothelial system including the lungs, liver, spleen and kidneys and forming first-generation schizonts. 5^{th} to 7^{th} days after infection, the subsequent crop of first-generation merozoites is released into the bloodstream, dispersing throughout the body and once again invading vascular endothelial cells and proliferating as second-generation schizonts which then separate from the host cells in the later stage and develop further in the intercellular spaces. On the 14^{th} day, second-generation merozoites from these schizonts are released into the blood, infiltrate and develop in the red blood cells, and are released from the host cells by the 18^{th} to 19^{th} day, maturing respectively into macrogametocytes and microgametocytes.

Both kinds of gametocytes enter the midgut of a Culicoides together with blood when the Culicoides sucks blood from the chicken, forming macrogametes and microgametes that are then fused (fertilized), passing through a tigote stage to become ookinetes which then infiltrate the intercellular spaces of the intestinal walls and then move under the outer membrane and develop into oocysts. Several sporozoites form inside each oocyst and move into the salivary glands, completing the developmental cycle.

The symptoms and pathology of chickens infected by this protozoon appear mainly the late stage of schizogony in the development of the second-generation schizonts and during the period of gametogony as the second-generation merozoites parasitize the red blood cells and develop into gametocytes. The hemorrhagic lesions of various organs and tissues and anemic symptoms that are most characteristic of this disease are attributed to bleeding accompanying vascular blockage caused by second-generation schizonts and to rupture of red blood cells due to parasitization by the protozoon in gametogony.

The following conventional methods are known for preventing and diagnosing Leucocytozoon disease of chickens.
(1) Prevention can be accomplished using the chemical agents pyrimethamine and sulfanilamide, and great success was achieved in controlling occurrence of the disease by the addition of chemical agents to feed, a method which became popular as a the cheapest and most reliable means of preventing epidemics. Subsequently, however, chemical-resistant protozoa appeared, followed by the development of improved chemicals and the emergence of more resistant protozoa, but when the feed safety rules were partially revised it was no longer possible to use drugs for laying hens.
(2) Since this disease is transmitted by Culicoides arakawae, the idea emerged of improving poultry house structures to keep out the midges, with windowless poultry houses being a typical strategy, but insect-proof screens and blowers have also been tried, sometimes in combination with insecticides.
(3) Two types of vaccines have been proposed, a live vaccine using sporozoites and an inactivated vaccine using a substance derived from insect bodies as the antigen. Both Culicoides and chickens are required to manufacture either of these vaccines, making them difficult to manufacture safely, cheaply and in mass quantities, so there is a limit on mass production on an industrial scale using conventional, low-productivity methods of vaccine manufacture.

Therefore, it has been proposed that in order to prevent Leucocytozoon disease without using chickens or Culicoides, a vaccine be prepared using a protein expressed by means of a recombinant prepared by genetic engineering methods, thereby providing genetically recombinant clones expressing a chicken Leucocytozoon immunogenic protein and a genetically recombinant vaccine for chicken Leucocytozoon disease which can be supplied safely, cheaply and in mass quantities. For example, one has been proposed (Japanese Patent Application Laid-open No. H7-284392) using an immunogenic protein gene clone, an immunogenic protein (R7) screened from a cDNA library prepared from mRNA coding for a protein found in second-generation schizonts of Leucocytozoon protozoa.

It has also been proposed (Japanese Patent Application Laid-open No. H7-89995) that a gene coding for a protein participating in phylaxis of a Leucocytozoon protozoon be cut out from the second-generation schizont of the Leucocytozoon protozoon by a gene recombination technique using the restriction enzymes Dral and HindIII as a DNA fragment which is then ligated with the expression system of an expression vector (pMAL-C) cut out with restriction enzymes StuI and HindIII to construct a plasmid (p24DH) so that a protein useful as a vaccine can be expressed in E. coli containing the resulting plasmid. However, vaccine manufacture and administration need to be made easier and more economical. No proposals can be found for immunogenic or functional peptides that are suited to peroral carrier systems, or for plants that produce such peptides.

Under these circumstances, and in an effort to solve the aforementioned problems in light of the aforementioned prior art, the inventors discovered, as a result of exhaustive research aimed at developing a target peptide with the function of resisting degradation in the digestive tract without being broken down before arriving at the intestines where its action is inteded, that it was possible to transport the target protein to the target site by orally administering a plant expressing a fused gene consisting of a gene coding for the target peptide and a gene coding for a Norwalk virus coat protein, so that the aforementioned problems could be solved by for example preparing a plant body expressing the LRE1 gene coding for the immunogenic peptide LRE1 derived from Leucocytozoon caulleryi second-generation schizonts, and perfected the present invention based on these findings.

### DISCLOSURE OF THE INVENTION

That is, it is an object of the present invention to provide a carrier system having the function of transporting a target peptide to a target site in an animal or human by orally administering a recombinant plant having a fused gene consisting of a gene coding for the target peptide and a gene coding for a Norwalk virus coat protein introduced and expressed therein. It is also an object of the present invention to provide a recombinant plant that produces the target peptide in such a way that even after oral administration the peptide resists decomposition in the digestive tract and reaches the intestinal tract or other destination where its action is intended.

Moreover, it is an object of the present invention to create a recombinant plant that produces an immunogenic peptide, blood pressure-lowering peptide or other functional peptide. It is also an object of the present invention to allow a plant that produces a target peptide to be cultivated in mass quantities so that the target peptide can be provided economically in mass quantities. It is also an object of the present invention to provide a peroral vaccine carrier system using a plant having the LRE1 gene coding for the immunogenic peptide LRE1 derived from the Leucocytozoon caulleryi second-generation schizont introduced so as to be expressed therein.

It is also an object of the present invention to allow easy and efficient vaccine administration without the stress caused be intramuscular injection by causing a human or animal to ingest the target peptide via a plant. It is also an object of the present invention to create a novel technique useful in the medical and pharmaceutical fields that allows production and oral administration of a target peptide by means of a plant.

To resolve the aforementioned issues, the present invention comprises the following technical means.
(1) A peroral carrier system having a function of transporting a target peptide with immunogenicity and functions to a target site in a living body by means of peroral administration, comprising
   as an active component a recombinant plant having an introduced fused gene obtained by fusing a gene coding for the target peptide with a Norwalk virus (NLV) coat protein gene and effecting expression of the target peptide.
(2) The carrier system for peroral administration of a target peptide according to (1) above, wherein the target peptide is an immunogenic peptide or functional peptide.
(3) The carrier system for peroral administration of a target peptide according to (2) above, wherein the immunogenic peptide is the immunogenic peptide LRE1 derived from the Leucocytozoon caulleryi 2^{nd} generation schizont.
(4) The carrier system for peroral administration of a target peptide according to (1) above, wherein the fused gene obtained by fusing a gene coding for the target peptide with a Norwalk virus coat protein gene is the recombinant gene represented by SEQ ID NO:1.
(5) The carrier system for peroral administration of a target peptide according to (1) above, wherein a potato as a recombinant plant having the introduced fused gene and effecting expression of the target peptide is used.
(6) The carrier system for peroral administration of a target peptide according to (1) above, wherein a target peptide extracted and refined from a recombinant plant having the introduced fused gene and effecting expression of the target peptide.
(7) A recombinant plant having a function of transporting a target peptide to a target site in a living body by oral administration, comprising
   a recombinant plant having an introduced fused gene obtained by fusing a gene coding for the target peptide with a Norwalk virus (NLV) coat protein gene and effecting expression of the target peptide in the plant body.
(8) The recombinant plant according to (7) above, wherein the target peptide is an immunogenic peptide or functional peptide.
(9) The recombinant plant according to (8) above, wherein the immunogenic peptide is the immunogenic peptide LRE1 derived from the Leucocytozoon caulleryi 2^{nd} generation schizont.
(10) The recombinant plant according to (7) above, wherein the fused gene obtained by fusing a gene coding for the target peptide with a Norwalk virus coat protein gene is the recombinant gene represented by SEQ ID NO:1.
(11) The recombinant plant according to (7) above, wherein the recombinant plant having the introduced fused gene and effecting expression of the target peptide in the plant body is a potato plant.
(12) A Calici-LRE1 antigen recombinant gene represented by SEQ ID NO:1, comprising LRE1 gene coding for the immunogenic peptide LRE1 derived from the Leucocytozoon caulleryi 2^{nd} generation schizont linked to a Norwalk virus coat protein gene.

The present invention is explained in more detail below.

The present invention relates to a carrier system for oral administration having the function of transporting a target peptide to a target site in a living body, wherein a plant expressing a fused gene obtained by fusing a gene coding for a target peptide such as an immunogenic peptide or functional peptide with a Norwalk virus coat protein gene is orally administered to a human or animal, and to a recombinant plant.

The present invention relates for example to a peroral vaccine carrier system in which a plant expressing a Calici-LRE1 antigen-expressing carrier system gene consisting of the LRE1 gene coding for the immunogenic peptide LRE1 derived from the Leucocytozoon caulleryi second-generation schizont fused with a Norwalk virus coat protein gene is orally administered to chickens, and to a potato or other recombinant plant to be used in this system. In the present invention, a gene coding for a target peptide is fused to a Norwalk virus coat protein gene, and this fused gene is introduced so as to be expressed in a plant body, thereby creating a recombinant plant that is orally administered so as to exert the useful function of the peptide.

The peroral carrier system of the present invention is implemented by ingestion of a genetically recombinant plant, and the desired object can be achieved merely by oral administration of the recombinant plant without the necessity of extracting or refining the target peptide. However, a target peptide produced by the recombinant plant may also be orally administered after having been extracted and separated by known methods.

The Norwalk virus is a virus that causes diarrhea symptoms in mammals, and its viral particles are not digested or broken down in the digestive tract, but arrive at the intestinal tract where they cause disease by acting on the intestinal mucosa. On the other hand, when peptides which are effective as vaccines are orally administered, because these peptides do not have similar resistance to decomposition in the digestive tract, they are broken down before they can arrive at the target site in the intestines or the like and exert their effects.

Thus, by fusing a target functional peptide with a viral particle (gene) associated with diarrhea and having resistance to decomposition in the digestive tract, a pseudo-viral particle is formed that resists decomposition and can therefore be transported to a target site (the intestinal mucosa or the like) without being broken down in the digestive tract, allowing the functional of the target peptide to be induced. Since in the present invention expression is achieved via fusion with a viral particle (gene) that causes diarrhea symptoms in mammals, it is effective for oral administration in mammals and also in birds.

In one example of a method for creating the transformed plant of the present invention, a ligated gene for plant expression, the Calici-LRE1 antigen gene comprising the LRE1 gene coding for the immunogenic peptide LRE1 derived from the Leucocytozoon caulleryi second-generation schizont linked to a Norwalk virus coat protein gene, is introduced and proliferated in E. coli. Next, the extracted plasmid is introduced into Agrobacterium, and a plant is infected with this transformed Agrobacterium, thereby introducing the antigen gene into the plant to create a transformed plant. However, a transformed plant can also be created in the same way using another target peptide.

Next, the present invention is explained in detail using the immunogenic peptide LRE1 derived from the Leucocytozoon caulleryi second-generation schizont as a typical example. The applicability of the present invention to mammals and birds is largely due to the properties of the Norwalk virus coat protein. The LRE1 gene coding for the immunogenic peptide LRE1 derived from the Leucocytozoon caulleryi second-generation schizont in the present invention is described in Japanese Patent Application Laid-open No. H7-284392, and the LRE1 peptide is the minimal protective epitope region of the R7 antigen. The Norwalk virus coat protein gene is described in Someya, Y., N. Takeda & T. Miyama, "Complete nucleotide sequence of the chiba virus genome and functional expression of the 3C-like protease in Escherichia coli", Virology, 2000, Dec. 20, 278(2):490-500.

These two genes were linked together as follows to construct the Calici-LRE1 gene. PCR was performed using primers designed as described below (Table 1) in order to fuse the Calici capsid protein deletion gene to the Leucocytozoon protozoon epitope gene and to add the SmaI restriction enzyme recognition site to the 5' end and the SacI restriction enzyme recognition site and plant ER retention signal peptide sequence (KDEL sequence) to the 3' end of this fused gene.

The SmaI restriction enzyme recognition site was deleted from the 5' end and 10 amino acids from the 3' end of the Calici capsid protein gene, and PCR was performed using primers (calici5sma, LRE1-14a.a.)(Table 1) designed to code for the epitope sequence of the Leucocytozoon protozoon along with LATaq^{™} (TaKaRa) and the GC buffer and reaction liquid included therewith. Following a thermal reaction consisting of 1 minute of heating at 94°C followed by 30 cycles of 30 seconds at 94°C, 30 seconds at 55°C and 2 minutes at 72°C, elongation was performed for 5 minutes at 72°C. The DNA fragment amplified by PCR was electrophoresed on 1% agarose gel, and a roughly 1.65 bp amplified band was confirmed, excised from the agarose gel, and purified.

Next, PCR was performed under the aforementioned conditions with the collected DNA fragment as the template using primers similar to those described above for the 5' end and primers designed to code for the epitope sequence, KDEL sequence and SacI restriction enzyme recognition site (calici5sma, LRE1-8aaKDELsac) (Table 1) for the 3' end along with LA Taq^{™} (TaKaRa) and the GC buffer and reaction liquid included therewith. The DNA fragment amplified by PCR was electrophoresed on 1% agarose gel, a roughly 1.7 bp band was confirmed, and the DNA fragment was purified.

The purified DNA fragment and a pUC118 vector (TaKaRa) were digested with the restriction enzymes SmaI and SacI (TaKaRa), and ligated to insert the DNA fragment into the pUC118 vector. E. coli JM109 (TaKaRa) competent cells were transformed with the entire amount of plasmid, and then seeded on LB solid medium containing ampicillin (50 µg/ml) and cultured overnight at 37°C to obtain a transformant strain. A plasmid was extracted from this transformant strain, and its nucleotide sequence was analyzed.

**[Table 1]**

| PRIMERS USED TO PREPARE PROTOZOAN ANTIGEN CARRIER SYSTEM GENE | |
|---|---|
| PRIMER | NUCLEOTIDE SEQUENCE |
| Calici5sma | 5'-TACCCGGGATGATGATGGCGTCTAAGGACG-3 |
| LRE1-14a.a. | |
| LRE1-8aaKDELSac | |

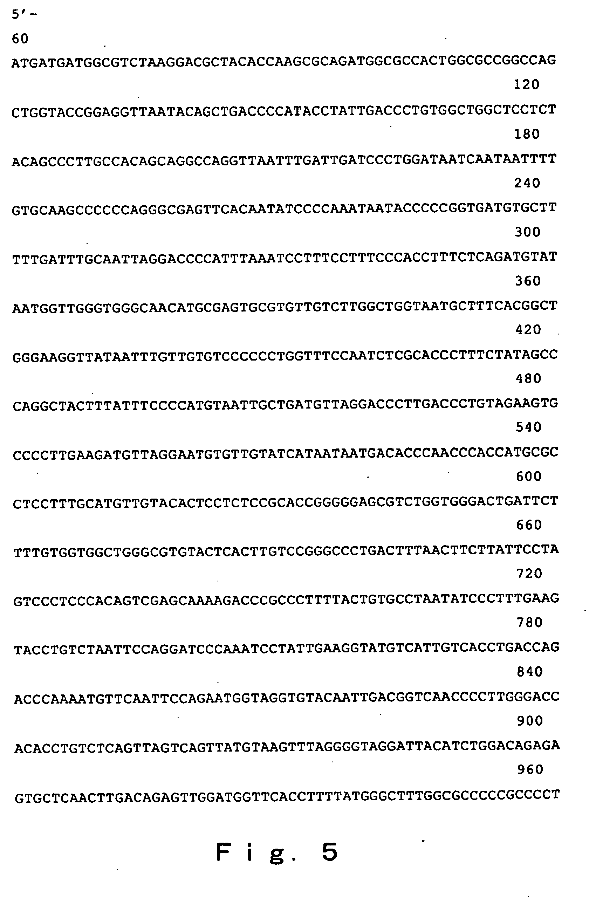

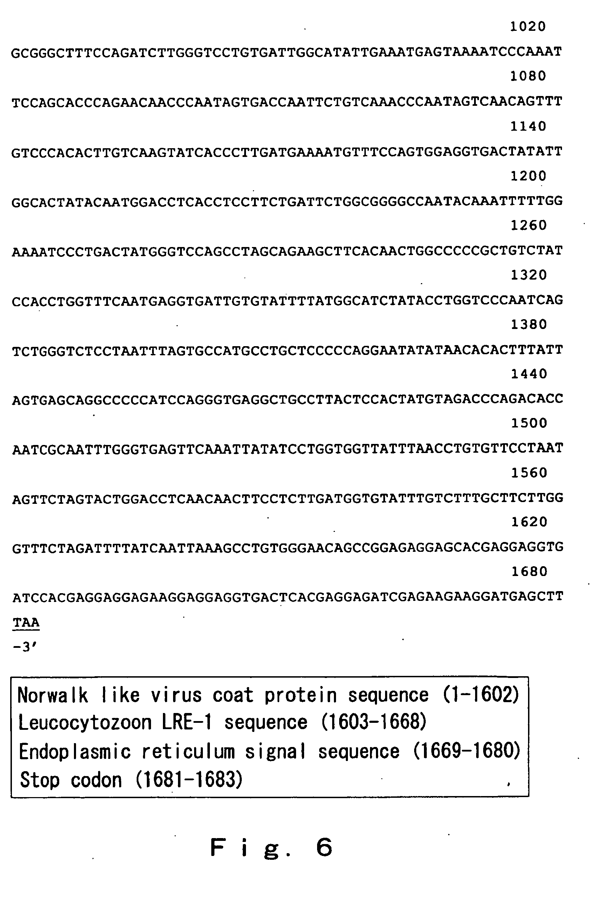

The sequence of the Calici-LRE1 gene is shown in Figures 5 and 6.

This Calici-LRE1 gene is inserted into a vector having a promoter. The promoter may be any known promoter suited to performing transcription within plant cells, but a desirable example is the cauliflower mosaic virus 35S promoter, although this is not a limitation.

The recombinant vector used in the present invention may be based on any vector capable of transforming a plant and replicating inside the plant cells. The vector preferably includes, in addition the aforementioned promoter, a replicator that functions within the cells as well as a terminator and a selection marker such as drug resistance. Such vectors are commercially available, and one example of a plant expression vector is pBE2113 having the 35S promoter of the cauliflower mosaic virus.

The recombinant vector used in the present invention can be easily prepared by using a restriction enzyme to insert the Calici-LRE1 gene into the aforementioned base vector by standard methods. Specific preparation methods are described in the following examples, but these examples are not limiting.

A preferred method for transforming the plant used in the present invention is to use the aforementioned recombinant vector to transform an Agrobacterium which is strongly infectious in plants, and infect a plant by inoculating it with the Agrobacterium, and a well-known transformation method using Agrobacterium can be used, such as freezing and thawing for example.

The plant used in the present invention is preferably one that produces the immunogenic LRE1 peptide or other target peptide in large quantities, such as a potato plant for example, but is not particularly limited. Moreover, when a potato plant is transformed the variety is not particularly restricted.

It is recognized by those skilled in the art that minor substitutions, deletions and insertions in DNA generally do not materially affect its function. Consequently, a plant can be transformed with the Calici-LRE1 gene sequence shown in the sequence tables and in Figures 5 and 6 even if that sequence has undergone minor substitutions, deletions or insertions, and it is still included in the fused gene of the present invention if it is still applicable to the carrier means of the present invention.

As explained above, the present invention relates to a carrier system for oral administration of a target peptide having as an active component a recombinant plant obtained by introducing and expressing in a plant body a fused gene obtained by fusing a gene coding for the target peptide with a Norwalk virus coat protein gene, and to that plant body, and relates to a new technique for creating a recombinant plant body expressing a target peptide and orally administering it to a human or animal, thereby causing the function of that peptide to be exerted. An example thereof is a transformed plant useful for producing antibodies for preventing disease of chickens caused by infection with a Leucocytozoon protozoon, along with an oral vaccine carrier system using that plant. This transformed plant expresses an immunogenic peptide found in second-generation schizonts, a strongly immunogenic stage in the life cycle of the protozoon, and antibodies can be produced in the chickens by orally administering this plant together with feed.

To overcome the problem of decomposition in the digestive tract that occurs with oral vaccines, the LRE1 gene coding for the immunogenic peptide LRE1 derived from the second-generation schizont of Leucocytozoon caulleryi is linked to a Norwalk virus coat protein gene to create a Calici-LRE1 immune expression gene that is introduced into plants, increasing its effectiveness as an oral vaccine. Moreover, the plant body having an immunogenic peptide of the present invention can be cultivated on a large scale to produce large quantities of a vaccine that is easy to administer, making it useful as a novel vaccine for preventing Leucocytozoon disease in chickens.

The present invention achieves the particular effects of (1) providing a carrier system having the function of transporting a target peptide to a target site in an animal or human by means of the oral administration of a recombinant plant having introduced and expressed therein a fused gene obtained by fusing a gene coding for the target peptide with a gene coding for a Norwalk virus coat protein, (2) providing a recombinant plant that produces a target peptide in such a way that even after oral administration the peptide resists decomposition in the digestive tract and reaches the intestinal tract or other destination where its action is intended, (3) creating a recombinant plant that produces an immunogenic peptide, blood pressure-lowering peptide or other target peptide, and allowing mass cultivation of a plant body that produces the target peptide so that the target peptide can be provided economically in large quantities, (4) allowing easy and efficient vaccine administration without the stress caused be intramuscular injection by causing a human or animal to ingest the target peptide via a plant, (5) creating a novel technique useful in the medical and pharmaceutical fields that allows production and oral administration of a target peptide by means of a plant.

### BRIEF DESCRIPTION OF THE DRAWINGS

Figure 1 shows the expressed amount of the Calici-LRE1 protein in a potato.
Figure 2 shows expression in transformed potato leaves confirmed by Western blotting.
Figure 3 shows Calici-LRE1 gene introduction in re-differentiated potato confirmed by genomic PCR.
Figure 4 shows antibody test results for a chicken fed with the potato of the present invention.
Figure 5 shows the first half of the Calici-LRE1 gene sequence.
Figure 6 shows the second half of the Calici-LRE1 gene sequence.

### BEST MODE FOR CARRYING OUT THE INVENTION

The present invention is explained in detail below based on examples, but the present invention is not in any way limited by these examples, and the present invention is applicable to immunogenic peptides, blood pressure-lowering peptides and other functional peptides in general, as well as to mammals and birds other than chickens.

### Example 1

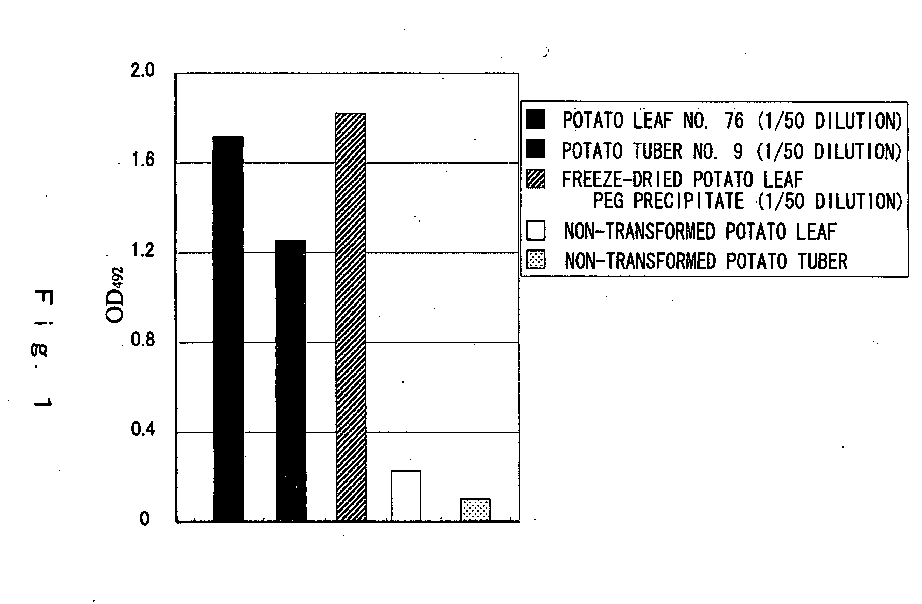

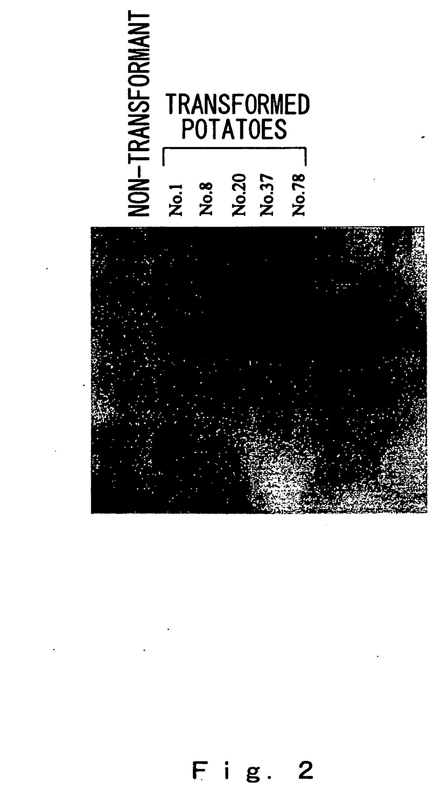

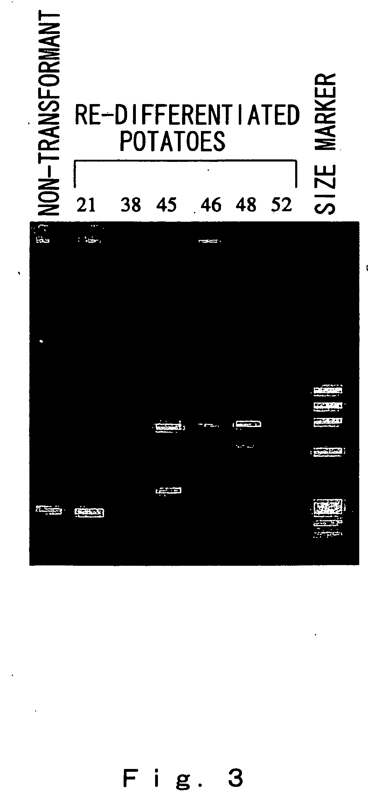

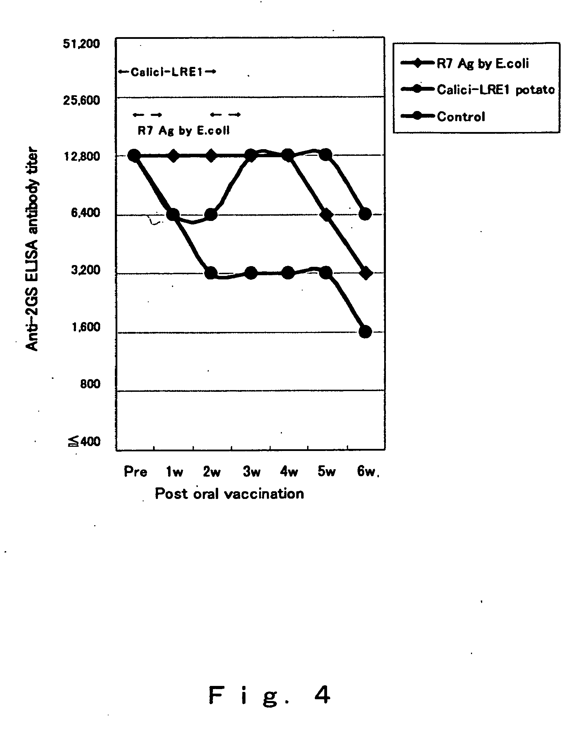

(1) Creation of potato having introduced Calici-LRE1 gene obtained by linking genes for immunogenic LRE1 protein derived from Leucytozoon caulleryi 2^{nd}-generation schizont and Norwalk virus coat protein
   The plant expression vector pBE2113 containing the cauliflower mosaic virus 35S promoter was digested at the SmaI and SacI sites, while the Calici-LRE1 gene (SEQ ID NO:1) obtained by linking the LRE1 gene coding for the LRE1 immunogenic peptide derived from the Leucocytozoon caulleryi 2^{nd}-generation schizont with the Norwalk virus coat protein gene was similarly digested at the SmaI and SacI sites, and pBE2113 and the Calici-LRE1 gene were ligated to construct pBE/Calici-LRE1, which was used in the following experiments as a kanamycin-resistant plant expression gene. PBE/Calici-LRE1 was introduced into E. coli (HB101), the E. coli was proliferated, and the aforementioned plasmid was extracted from the resulting transformed E. coli.
   The CAlici-LRE1 gene-introduced plasmid pBE/Calici-LRE1 obtained as described above was introduced into Agrobacterium tumefaciens LBA 4404 (Clontech) by the direct method described below using freezing and thawing. Agrobacterium tumefaciens LBA 4404 was shaking cultured at 28°C in 50 mL of LB liquid medium (1% Bacto Tryptone, 0.5% yeast extracts, 1% sodium chloride) until the A600 absorbance value was about 1.0. After being cooled on ice, this was centrifuged at 4°C, 300 g using a Kubota RA-6, and the cells were collected and floated in 1 mL of ice-cooled 20 mM calcium chloride solution. This was dispensed in amounts of 0.1 mL in Eppendorf tubes. 1 µg of the recombinant plasmid pBE/Calici-LRE1 was then added to the cells, which were then immediately frozen with liquid nitrogen.
   Next, the resulting frozen cells were thawed at 37°C, and left standing for 5 minutes. 1 mL of LB medium was added thereto, followed by shaking culture for 2 to 4 hours at 28°C. The cells were collected by centrifugation for 1 minute at about 10,000 g (using Kubota KM-15200), floated in 0.1 mL of LB medium, spread on LB solid medium containing rifampicin (100 µg/mL), kanamycin (25 µg/mL) and streptomycin (300 µg/mL), and agitated for 2 to 3 days at 28°C to obtain a transformed .bacteria incorporating pBE/Calici-LRE1.
   The Calici-LRE1 gene was introduced into a potato using the Agrobacterium prepared above and the tuber disk method. The potato transformation operation or in other words the infection test consisted of infection by the tuber disk method using the prepared Agrobacterium LBA4404 having pBE/CaliciLRE1. That is, a potato tuber was sterilized for 15 minutes in a 1% sodium hypochlorite solution and washed 2 to 3 times with sterile distilled water, a cylinder about 1 cm in diameter was punched out with a sterilizer cork puller, and a tuber disk about 2 mm in thickness was sliced from the cylinder using a knife.
   The Agrobacterium having the gene to be introduced was shaking cultured at 28°C in LB medium, cells were collected by centrifugation at 4°C, 8,000 rpm (Kubota KR-20000T) and suspended in MS liquid medium (pH 5.9, containing 3-indolacetic acid 50 µg/l, gibberellic acid 100 µg/l, abscisic acid 100 µg/l and zeatin-riboside 2 mg/l) and the disk was immersed in this liquid for 15 minutes. This was followed by co-culture for 3 days at 24 to 25°C on MS solid medium to infect the callus with the Agrobacterium, after which the callus was washed with MS liquid medium containing an antibiotic to remove the Agrobacterium. After washing, the callus was laid on MS solid medium containing an antibiotic, and subcultured every 2 weeks under conditions of 22°C, 16 hours light, 8 hours darkness to create a re-differentiated strain. After transformation, the re-differentiated individuals were transplanted to rooting medium.
(2) Confirming expression of Calici-LRE protein in genetically recombinant potato
   5 times the amount of PBS-T buffer (135 mM sodium chloride, 1.5 mM sodium dihydrogen phosphate, 2.7 mM disodium hydrogen phosphate, 0.05% (v/v) Tween 20, pH 7.2) was added to leaves of the re-differentiated strain obtained by introducing a gene for plant expression to prepare a sample that was made into a raw liquid. This material was subjected to ELISA testing using anti-Norwalk virus serum. Anti-Norwalk like virus serum (rabbit polyclonal antibodies from the National Institute of Infectious Diseases) or anti-Norwalk like virus antibodies (mouse monoclonal antibodies) were diluted with 0.05 M carbonate-bicarbonate buffer (15 mM sodium carbonate, 35 mM sodium bicarbonate, 2.5 mM sodium azide), added to an ELISA plate (Maxisorp; Nunc), and solidified on the plate for 3 hours at 37°C.
   The solidified plate was washed 3 times with PBS-Tween buffer, and the prepared raw liquid of leaves and seeds of the recombinant plant body was added and incubated overnight at 4°C. This plate was washed 3 times with PBS-Tween buffer, and anti-NLV HRPO conj. was added successively. This plate was washed 3 times with PBS-Tween buffer, and o-phenylenediamine was added as a substrate and reacted at room temperature for 30 minutes. The reaction was stopped with a stop solution (5% sodium lauryl sulfate), and absorbance at 493 nm was measured with a microplate reader (Bio Rad model 2550 EIA reader).
   As a result, some of the strains in the tests were confirmed to have a much higher level of expression than the non-recombinant (Figure 1). Individuals were selected of 7 strains having a high level of expression of the protozoan antigen carrier system protein in their leaves. Samples were prepared as before with the leaves, and an ELISA test was performed on the tubers. As a result, a high level of expression was confirmed in the tubers as it was in the leaves (Figure 1).
   Expression was confirmed by Western blotting using a raw extract of leaves of the re-differentiated strain used in the ELISA test. That is, SDS-polyacrylamide gel electrophoresis (PAGE) sample buffer was added to the raw plant liquid, which was then heated for 3 minutes in boiling water to denature the proteins. 20 µl of this solution was electrophoresed by the Laemmli method (Laemmli, 1970) using 8% SDS-polyacrylamide gel. After electrophoresis, the gel was rinsed with distilled water, and the proteins were transferred to an ECL membrane (Amersham) by standard methods using a Mini-Protean II (Bio-Rad). After transfer, the membrane was washed with ethanol and equilibrated by being immersed overnight in Block-Ace.
   Anti-Norwalk like virus serum was diluted with TBS buffer (20 mM Tris-HCl, 0.14 M NaCl, pH 7.5), and the equilibrated membrane was agitated for 1 hour in this solution. After agitation, the membrane was rinsed 3 times with TBS-T buffer (TBS buffer-0.1% (v/v) Tween 20), and agitated for 1 hour with HPRO-labeled antibodies diluted with TBS-T buffer. After the reaction the membrane was washed 5 times with TBS-T buffer, and chemiluminesced with ECL-Plus (Amersham). Luminescence was detected with Versa Doc (Bio Rad). The raw extract of the re-differentiated individuals was fractioned by SDS-PAGE, and reacted with anti-NLV serum. As a result, the anti-NLV serum did not react with the non-recombinant form, but only reacted with the re-differentiated strains having the introduced protozoan antigen carrier system gene (Figure 2).
(3) Confirming introduction of Calici-LRE1 gene into potato body
   Gene introduction of the Calici-LRE1 gene into the potato was confirmed by genomic PCR. Total DNA extract from potato leaves was used as well as a RED Extract-N-AmpTMPlant PCR Kit (SIGMA) for the genomic PCR. Using total DNA extracted from leaves of the prepared re-differentiated strains as the template, two kinds of primers ((CaL553:5'-TTGTACACTCCTCTC-3' : Seq 2) and (CaL1423:5'-AGTGGAGTAAGGCAG-3': Seq 3) were designed based on the nucleotide sequence of the Calici-LRE1 gene, and used in genomic PCR. That is, using total DNA (4 µl) of the extracted plant as the template, the total volume was adjusted to 20 µl with DW, and PCR was performed using primers designed based on the nucleotide sequence of the introduced gene (final concentration 0.4 µM) and REDExtract-N-Amp PCR reaction mix (10 µl).
   The PCR reaction consisted of 3 minutes at 94°C followed by 30 cycles of a cycle consisting of 30 seconds at 94°C, 30 seconds at 55°C and 1.5 minutes at 72°C, followed by an elongation reaction of 10 minutes at 72°C. A thermal cycler (MJ Research DNA Engine PTC-200) was used for this reaction. As a result, a band derived from the re-differentiated strain appeared at position of the target size (about 870 bp) in the agarose gel electrophoresis pattern. No band was confirmed at this location using the non-recombinant potato as a control (Figure 3). These results confirm introduction of the protozoan antigen carrier system gene into the potato.
(4) Confirming antigenicity of Calici-LRE1 gene-introduced potato
   Leaves of a potato having the introduced Calici-LRE1 gene were freeze-dried to a dried weight of about 1/12 the fresh weight in a 48-hour process using a Kyowa Vacuum Engineering RLE-204. The dried leaves were crushed into a powder and mixed with formula feed for chickens (Funabashi Farms) to make a material for oral administration.
   The chickens used in the oral administration test were injected with a chicken Leucocytozoon disease vaccine widely used in the field (using as the vaccine antigen component a R7 protein derived from the Leucoytozoon caulleryi second-generation schizont and expressed in E. coli (Japanese Patent Application Laid-open No. H07-284392)), and three individuals having anti-2Gs ELISA antibody titer of 12,800 times were assigned to oral administration of freeze-dried leaves of Calici-LRE1 antigen-expressing potato, oral administration of R7 antigen expressed in E. coli (freeze-dried and powdered as in the case of the plant material), which was the injected vaccine antigen, and no oral administration.
   Freeze-dried potato leaves expressing the Calici-LRE1 antigen were given in a total of 60 g of feed per chicken per day (with about 6.7 g being leaves of Calici-LRE1 gene-introduced potato, for a daily antigen dose of about 1,500 units of antigen titer, and a total administered antigen volume of about 20,000 units per chicken), and orally administered continuously for 13 days (ingested voluntarily). Upon completion of feeding including leaves of Calici-LRE1 gene-introduced potato, each chicken was given 60 g of formula feed for chickens (Funabashi Farms).
   The R7 antigen expressed in E. coli was also given by adding R7 antigen powder in a total of 60 g of feed per chicken per day so that 1,000,000 units of antibody titer were included in the feed, and administered orally for 7 days, followed by 7 days without administration and 7 days of continuous administration (for a total administered antigen volume of 14,000,000 units per chicken). 60 g per day of ordinary formula feed for chickens was also administered to 1 control chicken from the beginning of testing. Blood was taken from the under-wing veins of all test chickens including the control chicken periodically after ingestion of the orally administered material, and centrifuged to obtain serum. This serum was used in the following antibody testing by ELISA.
   Leucocytozoon caulleryi second-generation schizont ultrasound solubilized antigen (Ito, A. et al., J. Vet. Med. Sci. 64(5):405-411, 2002) diluted to a concentration of 0.1 µg/mL with 0.05 M sodium carbonate buffer (1.59 g disodium carbonate, 2.93 g sodium bicarbonate per liter, pH 9.6) was distributed on a 96-well ELISA plate (Iwaki), and left to coat overnight at 4°C. The coated plate was washed with PBS-T buffer, and a blocking liquid consisting of bovine serum albumin diluted with PBS-T buffer to a final concentration of 3% (w/v) was poured onto the plate and left for 1 hour at 37°C to block the plate. After blocking, this was washed with PBS-T buffer, and serum diluted in 2 times stages from 400 times to 51,200 times with an antigen dilution liquid consisting of bovine serum albumin added to a final concentration of 0.3% (w/v) to PBS-T buffer was poured into the plate and left for 1 hour at 37°C to react with the antibodies.
   This plate was washed with PBS-T buffer, and HRPO-labeled anti-chicken IgG (ZYMED) diluted 12,000 times with antibody dilution liquid was distributed on the plate and reacted for 1 hour at 37°C. This plate was washed with PBS-T buffer, and a substrate solution (comprising 14.6 g of disodium hydrogenphosphate, 10.2 g of citric acid hydrate, 1 g of o-phenylenediamine and 1 mL of hydrogen peroxide solution per 1 L) was added to the plate and left to react in a dark place for 15 minutes at 37°C. The reaction was stopped with a stop solution (5 N aqueous sulfuric acid solution), and absorbance at A492 was measured with a microplate reader (Corona MTP-120 was used).
   The results of this test are shown in Figure 4. The antibody titer of the chicken receiving no oral administration declined rapidly after the beginning of the test, falling to 1,600 times at the 6^{th} week. In the chicken receiving oral administration of potato leaves expressing the Calici-LRE1 antigen, antibody titer dropped during oral administration, but rose after completion of oral administration, and was 6,400 times even at the 6^{th} week. In the chicken receiving oral administration of the R7 antigen expressed in E. coli (see Japanese Patent Application Laid-open No. H07-284392), the antibody titer also bottomed out, but then continued to decline gradually after completion of administration, falling to 3,200 times at the 6^{th} week. These test results show that in the chicken receiving oral administration of potato leaves expressing the Calici-LRE1 antigen, greater antibody re-induction was achieved even with an administered amount of antigen 1/700 that of the E. coli expressed R7 antigen, indicating the effectiveness of this carrier system or in other words of an oral immune material comprising a Norwalk virus coat protein and an exogenous antigen.

### INDUSTRIAL APPLICABILITY

As explained above, the present invention relates to a carrier means for transporting a target peptide to a target site in the body wherein a plant having introduced and expressed therein a fused gene obtained by fusing a gene coding for a target peptide with a Norwalk virus coat protein gene is orally administered, and allows the problems or prior art to be solved for example by means of a recombinant plant body expressing a functional or immunogenic peptide such as the immunogenic peptide LRE1 derived from the Leucocytozoon caulleryi second-generation schizont.

The present invention also provides a recombinant plant body that produces a functional peptide in such a way that when orally administered, the functional peptide resists degradation in the digestive tract and arrives at the intestines or other site where it is to act. A genetically recombinant plant is created that produces a target peptide such as an immunogenic peptide, blood pressure-lowering peptide or the like, and by mass cultivating this plant that produces a functional peptide, it is possible to provide the target peptide economically and in mass quantities, so that by for example orally administering to chickens a plant body expressing the LRE1 gene coding for the immunogenic peptide LRE1 derived from the Leucocytozoon caulleryi second-generation schizont, it is possible to provide an effective oral vaccine carrier system which is effective for preventing Leucocytozoon disease of chickens.

The present invention allows easy and efficient vaccine administration without the stress of intramuscular injection because a plant body expressing the aforementioned gene is administered to chickens in the form of feed. Moreover, the present invention is useful because it allows the creation of novel techniques to allow the production and oral administration of functional peptides which are useful in the medical and pharmaceutical fields.

## Claims

1. A peroral carrier system having the function of transporting a target peptide to a target site in a living body by means of peroral administration, comprising:
as an active component, a recombinant plant having a trans-gene comprising a gene coding for the target peptide fused with a Norwalk virus (NLV) coat protein gene and expressing said trans-gene, wherein the target peptide is the immunogenic peptide LRE1 derived from the Leucocytozoon caulleryi 2^{nd} generation schizont.

2. The carrier system for peroral administration of a target peptide according to Claim 1, wherein the trans-gene is the recombinant gene presented by SEQ ID NO:1.

3. The carrier system for peroral administration of a target peptide according to Claim 1, wherein said recombinant plant is a potato.

4. The carrier system for peroral administration of a target peptide according to Claim 1, wherein a target peptide is extracted and refined from said recombinant plant.

5. A recombinant plant having the function of transporting a target peptide to a target site in a living body by oral administration, said recombinant plant having a trans-gene comprising a gene coding for the target peptide fused with a Norwalk virus (NLV) coat protein gene and expressing said trans-gene, wherein the target peptide is the immunogenic peptide LRE1 derived from the Leucocytozoon caulleryi 2^{nd} generation schizont.

6. The recombinant plant according to Claim 5, wherein the trans-gene is the recombinant gene represented by SEQ ID NO:1.

7. The recombinant plant according to Claim 5, wherein the plant is a potato plant.

8. A Calici-LRE1 antigen recombinant gene represented by SEQ ID NO:1, comprising LRE1 gene coding for the immunogenic peptide LRE1 derived from the Leucocytozoon caulleryi 2^{nd} generation schizont linked to a Norwalk virus coat protein gene.

## Patentansprüche

1. Perorales Trägersystem, welches die Funktion hat, ein Target-Peptid zu einer Target-Stelle in einem lebenden Körper mit Hilfe einer peroralen Darreichung zu befördern, aufweisend:
als eine aktive Komponente eine rekombinante Pflanze, die über ein Transgen verfügt, das ein Gen aufweist, welches auf das Target-Peptid codiert, das mit einem Norwalk-Virus (NLV)-Hüllprotein-Gen fusioniert ist, und welches das Transgen exprimiert, wobei das Target-Peptid das immunogene Peptid LRE1 ist, das deriviert ist von dem Leukozytoon Caulleryi der zweiten Generation des Schizont.

2. Trägersystem für die perorale Darreichung eines Target-Peptids nach Anspruch 1, wobei das Transgen das rekombinante Gen ist, welches dargestellt wird durch SEQ ID No 1.

3. Trägersystem für die perorale Darreichung eines Target-Peptids nach Anspruch 1, wobei die rekombinante Pflanze eine Kartoffel ist.

4. Trägersystem für die perorale Darreichung eines Target-Peptids nach Anspruch 1, wobei ein Target-Peptid aus der rekombinanten Pflanze extrahiert und gereinigt wird.

5. Perorales Trägersystem, welches die Funktion hat, ein Target-Peptid zu einer Target-Stelle in einem lebenden Körper mit Hilfe einer peroralen Darreichung zu transportieren, wobei die rekombinante Pflanze, die über ein Transgen verfügt, das ein Gen aufweist, welches auf das Target-Peptid codiert, das mit einem Norwalk-Virus (NLV)-Hüllprotein-Gen fusioniert ist, und welches das Transgen exprimiert, wobei das Target-Peptid das immunogene Peptid LRE1 ist, das deriviert ist von dem Schizont der zweiten Generation von Leukozytoon Caulleryi.

6. Rekombinante Pflanze nach Anspruch 5, wobei das Transgen das rekombinante Gen ist, welches dargestellt wird durch SEQ ID No 1.

7. Rekombinante Pflanze nach Anspruch 5, wobei die rekombinante Pflanze eine Kartoffelpflanze ist.

8. Calici-LRE1-Antigen, dargestellt durch SEQ ID No 1, aufweisend LRE1-Gen, codierend auf das immunogene Peptid LRE1 ist, das deriviert ist von dem Leukozytoon Caulleryi der zweiten Generation des Schizont, das verknüpft ist mit einem Norwalk-Virus-Hüllprotein-Gen.

## Revendications

1. Système de vecteur peroral ayant pour fonction de transporter un peptide cible vers un site cible dans un organisme vivant par administration perorale, comprenant :
comme composant actif, une plante recombinée ayant un transgène comprenant un gène codant le peptide cible fusionné avec un gène de protéine d'enveloppe de virus de Norwalk (NLV) et exprimant ledit transgène, où le peptide cible est le peptide immunogène LRE1 provenant du schizonte de 2ème génération de Leucocytozoon caulleryi.

2. Système de vecteur pour l'administration perorale d'un peptide cible selon la revendication 1, où le transgène est le gène recombiné représenté par SEQ ID NO :1.

3. Système de vecteur pour l'administration perorale d'un peptide cible selon la revendication 1, où ladite plante recombinée est une pomme de terre.

4. Système de vecteur pour l'administration perorale d'un peptide cible selon la revendication 1, où un peptide cible est extrait et mis au point à partir de ladite plante recombinée.

5. Plante recombinée ayant pour fonction de transporter un peptide cible vers un site cible dans un organisme vivant par administration orale, ladite plante recombinée ayant un transgène comprenant un gène codant le peptide cible fusionné avec un gène de protéine d'enveloppe de virus de Norwalk (NLV) et exprimant ledit transgène, où le peptide cible est le peptide immunogène LRE1 provenant du schizonte de 2ème génération de Leucocytozoon caulleryi.

6. Plante recombinée selon la revendication 5, où le transgène est le gène recombiné représenté par SEQ ID NO :1.

7. Plante recombinée selon la revendication 5, où la plante est un plan de pomme de terre.

8. Gène recombiné d'antigène Calici-LRE1 représenté par SEQ ID NO:1 , comprenant le gène LRE1 codant le peptide immunogène LRE1 provenant du schizonte de 2ème génération de Leucocytozoon caulleryl lié à un gène de protéine d'enveloppe de virus de Norwalk.


### SEQUENCE LISTING

<110> Naitional Institute of Advanced Industrial Science and Technology
   Frontier Science Co., Ltd.
   The Kitasato Institute
   Hokkaido Green-Bio Institute
<120> Oral vaccine carrier system
<130> 200506F-0507
<150> JP2004-169259
   <151> 2004-06-07
<160> 1
<170> PatentIn version 3.1
<210> 1
   <211> 1683
   <212> DNA
   <213> Leucocytozon Caulleryi
<400> 1
   atgatgatgg cgtctaagga cgctacacca agcgcagatg gcgccactgg cgccggccag      60
   ctggtaccgg aggttaatac agctgacccc atacctattg accctgtggc tggctcctct     120
   acagcccttg ccacagcagg ccaggttaat ttgattgatc cctggataat caataatttt     180
   gtgcaagccc cccagggcga gttcacaata tccccaaata atacccccgg tgatgtgctt     240
   tttgatttgc aattaggacc ccatttaaat cctttccttt cccacctttc tcagatgtat     300
   aatggttggg tgggcaacat gcgagtgcgt gttgtcttgg ctggtaatgc tttcacggct     360
   gggaaggtta taatttgttg tgtcccccct ggtttccaat ctcgcaccct ttctatagcc     420
   caggctactt tatttcccca tgtaattgct gatgttagga cccttgaccc tgtagaagtg     480
   ccccttgaag atgttaggaa tgtgttgtat cataataatg acacccaacc caccatgcgc     540
   ctcctttgca tgttgtacac tcctctccgc accgggggag cgtctggtgg gactgattct     600
   tttgtggtgg ctgggcgtgt actcacttgt ccgggccctg actttaactt cttattccta     660
   gtccctccca cagtcgagca aaagacccgc ccttttactg tgcctaatat ccctttgaag     720
   tacctgtcta attccaggat cccaaatcct attgaaggta tgtcattgtc acctgaccag     780
   acccaaaatg ttcaattcca gaatggtagg tgtacaattg acggtcaacc ccttgggacc     840
   acacctgtct cagttagtca gttatgtaag tttaggggta ggattacatc tggacagaga     900
   gtgctcaact tgacagagtt ggatggttca ccttttatgg gctttggcgc ccccgcccct    960
   gcgggctttc cagatcttgg gtcctgtgat tggcatattg aaatgagtaa aatcccaaat   1020
   tccagcaccc agaacaaccc aatagtgacc aattctgtca aacccaatag tcaacagttt   1080
   gtcccacact tgtcaagtat cacccttgat gaaaatgttt ccagtggagg tgactatatt   1140
   ggcactatac aatggacctc acctccttct gattctggcg gggccaatac aaatttttgg   1200
   aaaatccctg actatgggtc cagcctagca gaagcttcac aactggcccc cgctgtctat   1260
   ccacctggtt tcaatgaggt gattgtgtat tttatggcat ctatacctgg tcccaatcag   1320
   tctgggtctc ctaatttagt gccatgcctg ctcccccagg aatatataac acactttatt   1380
   agtgagcagg cccccatcca gggtgaggct gccttactcc actatgtaga cccagacacc   1440
   aatcgcaatt tgggtgagtt caaattatat cctggtggtt atttaacctg tgttcctaat   1500
   agttctagta ctggacctca acaacttcct cttgatggtg tatttgtctt tgcttcttgg   1560
   gtttctagat tttatcaatt aaagcctgtg ggaacagccg gagaggagca cgaggaggtg   1620
   atccacgagg aggagaagga ggaggtgact cacgaggaga tcgagaagaa ggatgagctt   1680
   taa                                                                  1683